# EUROPEAN PATENT APPLICATION

(11) **EP 4 190 385 A1**
(43) Date of publication of application: **07.06.2023**
(21) Application number: 21853515.1
(22) Date of filing: 26.03.2021
(51) Int. Cl.: A61M 25/00

(54) **CATHETER AND METHOD FOR ENGAGING CATHETER**

(30) Priority: 03.08.2020 JP 2020131419; 30.11.2020 JP 2020197871
(71) Applicant: TERUMO Kabushiki Kaisha, Tokyo 151-0072 (JP)
(72) Inventor: OKAMURA Ryo, Ashigarakami-gun, Kanagawa 259-0151 (JP); YASUNAGA Mitsuteru, Fujinomiya-shi, Shizuoka 418-0015 (JP)
(74) Representative: Casalonga
(86) International application number: PCT/JP2021/012977
(87) International publication number: WO 2022/030046

(57) **Abstract**

[Problem] Provided is a catheter excellent in operability and a method for catheter engagement.

[Solution] A catheter (1) for a hepatic artery to be introduced from an arm of a patient includes a tubular body (2). The tubular body (2) includes an inner layer (31), an outer layer (32), and a reinforcement body (33) embedded in the tubular body (2). The tubular body (2) includes a substantially linear main body portion (23) and a shaped portion (24) shaped in a manner of being bent on substantially the same plane. The shaped portion (24) includes a first bent portion (25) defining a first angle (θ4) distal of the main body portion (23), a second bent portion (26) defining a second angle (θ5) distal of the first bent portion (25) and bent to the same side as the first bent portion (25), a third bent portion (27) defining a third angle (θ3) distal of the second bent portion (26) and bent to a side opposite to the second bent portion (26), and a most distal portion (28) disposed distal of the third bent portion (27). A physical property changing point is provided between a proximal portion (25A) of the first bent portion (25) and a distal portion (26A) of the second bent portion (26).

## Description

### Technical Field

The present invention relates to a catheter to be inserted into a body lumen and a method for engaging a catheter with a blood vessel.

### Background Art

Currently, intervention is performed in which treatment of lesion areas such as a heart, a blood vessel, a liver, a brain, a digestive organ, and a urologic is performed by an elongated catheter inserted into a blood vessel from a hole opened in a skin.

In recent years, a method of performing treatment by inserting a catheter from a radial artery of a wrist using trans radial intervention (TRI) has been widely performed (see, for example, PTLs 1 and 2). Introduction of the catheter from an artery of an arm has an effect of reducing a physical burden on a patient, accelerating hospital discharge, and the like.

When a lesion area is present in a liver, a treatment of supplying an embolic agent or a drug to the lesion area may be performed by a treatment catheter inserted to the vicinity of a hepatic artery. In such a treatment, it is desirable to use a guiding catheter such that the treatment catheter is capable of fully exhibiting a function at a target position.

### Citation List

### Patent Literature

PTL 1: Europe Patent No. 3013404
PTL 2: WO2015/146408

### Summary of Invention

### Technical Problem

As the guiding catheter for the hepatic artery introduced from the artery of the arm, for example, a guiding catheter for a coronary artery or the like may be used. However, the coronary artery and the hepatic artery have different shapes, and thus are difficult to operate. The same applies when being used for a case other than the hepatic artery.

The guiding catheter introduced from the artery of the arm is preferably easy to access a descending aorta from a subclavian artery. Further, it is preferable that the guiding catheter alleviates a reaction generated by insertion of the treatment catheter or a guide wire and applies a backup force to the medical device such as the treatment catheter or the guide wire so as to hold the treatment catheter at a desired position.

The invention has been made to solve the above-described technical problem, and an object thereof is to provide a catheter excellent in operability and a method for catheter engagement.

### Solution to Problem

In order to solve the technical problem, an aspect of the invention is a catheter for a hepatic artery to be introduced from an arm of a patient. The catheter includes a tubular body extending from a proximal end to a distal end. The tubular body includes an inner layer, an outer layer, and a reinforcement body embedded in the tubular body. The tubular body includes a substantially linear main body portion and a shaped portion shaped in a manner of being bent on substantially the same plane. The shaped portion includes a first bent portion defining a first angle distal of the main body portion, a second bent portion defining a second angle distal of the first bent portion and bent to the same side as the first bent portion, a third bent portion defining a third angle distal of the second bent portion and bent to a side opposite to the second bent portion, and a most distal portion disposed distal of the third bent portion. A physical property changing point is provided between a proximal portion of the first bent portion and a distal portion of the second bent portion.

### Advantageous Effect of Invention

In the catheter configured as described above, since the physical property changing point is disposed between the proximal portion of the first bent portion and the distal portion of the second bent portion, a position of the shaped portion of the catheter is stabilized by the first bent portion, which has a high strength, coming into contact with the descending aorta. Since the second bent portion which is at least partially flexible is engaged with the celiac artery in a favorable manner, and the third bent portion which is flexible and disposed distal of the physical property changing point is bent to the side opposite to the second bent portion, the most distal portion is easily directed to the common hepatic artery. Therefore, the catheter can apply a favorable backup force to another medical device inserted into the catheter. Further, since the shaped portion provided with the physical property changing point is likely to firmly maintain the shape proximal of the physical property changing point, an access of the catheter to the descending aorta is facilitated. Therefore, operability of the catheter is improved.

The physical property changing point may be disposed in the first bent portion. Accordingly, a distal side of the catheter can be stably directed toward the celiac artery by bending of the first bent portion which has a high strength, and the second bent portion which is flexible and disposed distal of the physical property changing point can be deformed in accordance with the celiac artery to be deeply engaged with the celiac artery (deep engage). Therefore, the catheter can apply a favorable backup force to another medical device inserted into the catheter. Since the second bent portion is flexible, it is possible to reduce a burden on a blood vessel wall in contact therewith.

The physical property changing point may be disposed in the second bent portion. Accordingly, since strengths of the first bent portion and the second bent portion are improved, a range in which the shape of the shaped portion can be firmly maintained is widened, and an access of the catheter from the subclavian artery to the descending aorta is facilitated.

The physical property changing point may be disposed between the first bent portion and the second bent portion. Accordingly, the distal side of the catheter can be stably directed toward the celiac artery by the bending of the first bent portion which has a high strength, and the second bent portion which is flexible and disposed distal of the physical property changing point can be deformed in accordance with the celiac artery to be deeply engaged with the celiac artery. Therefore, the catheter can apply a favorable backup force to another medical device inserted into the catheter. Since the second bent portion is flexible, it is possible to reduce the burden on the blood vessel wall in contact therewith. Further, since the strength of the entire first bent portion is improved, the range in which the shape of the shaped portion can be firmly maintained is widened, and the access of the catheter from the subclavian artery to the descending aorta is facilitated.

The physical property changing point may be an end portion of the reinforcement body. Since the reinforcement body enhances the hardness, it is possible to easily provide the physical property changing point without changing a resin of the inner layer or the outer layer.

When the second bent portion is disposed in contact with a blood vessel wall of the celiac artery, the most distal portion may be directed to the common hepatic artery, and the first bent portion may be in contact with a wall surface of the descending aorta on a side opposite to the celiac artery. Accordingly, the second bent portion can be engaged with the celiac artery in a favorable manner in a state in which the first bent portion, which has a high strength, is stably brought into contact with the blood vessel wall of the descending aorta on the side opposite to the celiac artery.

In order to solve the technical problem, another aspect of the invention is a method for catheter engagement. The method includes: a step of inserting, into an artery of an arm of a patient, a catheter including a tubular body extending from a proximal end to a distal end, the tubular body including an inner layer, an outer layer, and a reinforcement body embedded in the tubular body, the tubular body including a substantially linear main body portion and a shaped portion shaped in a manner of being bent on substantially the same plane, the shaped portion including a first bent portion disposed distal of the main body portion, a second bent portion disposed distal of the first bent portion and bent to the same side as the first bent portion, a third bent portion disposed distal of the second bent portion and bent toward a side opposite to the second bent portion, and a most distal portion disposed distal of the third bent portion, and a physical property changing point being provided between a proximal portion of the first bent portion and a distal portion of the second bent portion; a step of advancing the shaped portion of the catheter to a main artery accessible from the artery of the arm; a step of advancing the shaped portion to a first blood vessel coupled in a direction intersecting a long axis of the main artery; and a step of bringing the second bent portion into contact with a blood vessel wall of the first blood vessel and attaching the first bent portion to a blood vessel wall of the main artery on a side opposite to the first blood vessel to direct the most distal portion to a second blood vessel selected from at least two blood vessels bifurcated on a peripheral side of the first blood vessel.

In the method for catheter engagement configured as described above, the position of the shaped portion of the catheter can be stabilized by bringing the first bent portion, which has a high strength, into contact with the descending aorta. The second bent portion which is at least partially flexible can be engaged with the first blood vessel in a favorable manner, and the most distal portion can be directed toward the second blood vessel. Therefore, a surgeon can easily insert another medical device into the second blood vessel using the catheter as a guide.

### Brief Description of Drawings

FIG. 1 is a schematic diagram showing an arrangement of a catheter in a blood vessel.
FIG. 2 is a plan view showing a distal portion of the catheter according to an embodiment.
FIG. 3 is a plan view showing the distal portion of the catheter according to the embodiment.
FIG. 4 is a cross-sectional view showing a part of the catheter according to the embodiment.
FIG. 5 is a plan view showing a three-point bending tester.
FIG. 6 is a schematic diagram showing a state in which the catheter is inserted into a descending aorta.
FIG. 7 is a schematic diagram showing a state in which the catheter is inserted into a celiac artery.
FIG. 8 is a plan view showing a position of an end portion of a reinforcement body which is a physical property changing point, where (A) is a first modification and (B) is a second modification.
FIG. 9 is an overall view of a catheter according to Example 1 in which a part thereof in a longitudinal direction is omitted.
FIG. 10 is an overall view of a catheter according to Example 2 in which a part thereof in a longitudinal direction is omitted.

### Description of Embodiments

Embodiments according to the invention will be described hereinafter with reference to the drawings. Note that for convenience of explanation, dimensions in the drawings may be exaggerated and may be different from actual dimensions. Further, in the present specification and the drawings, structural elements that have substantially the same function are denoted with the same reference numerals, and repeated explanation of these structural elements is omitted. In the present description, a side to be inserted into a lumen is referred to as a "distal side", and a side to be operated is referred to as a "proximal side".

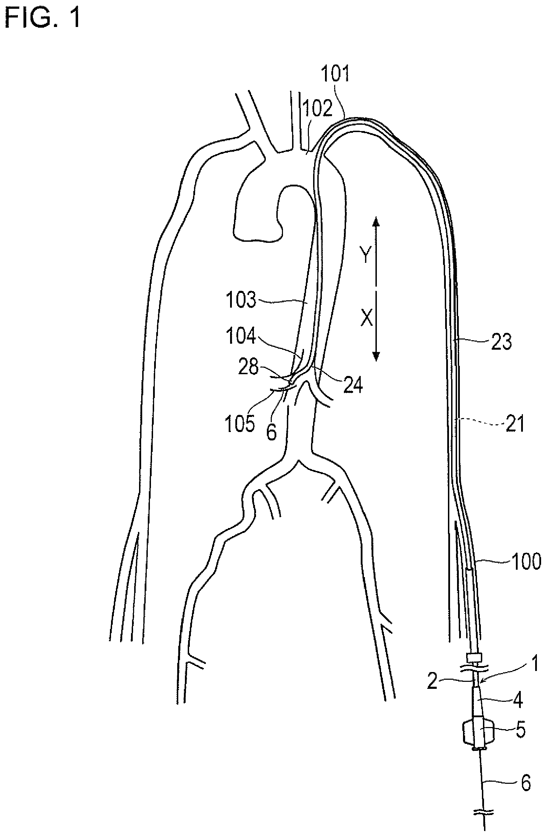

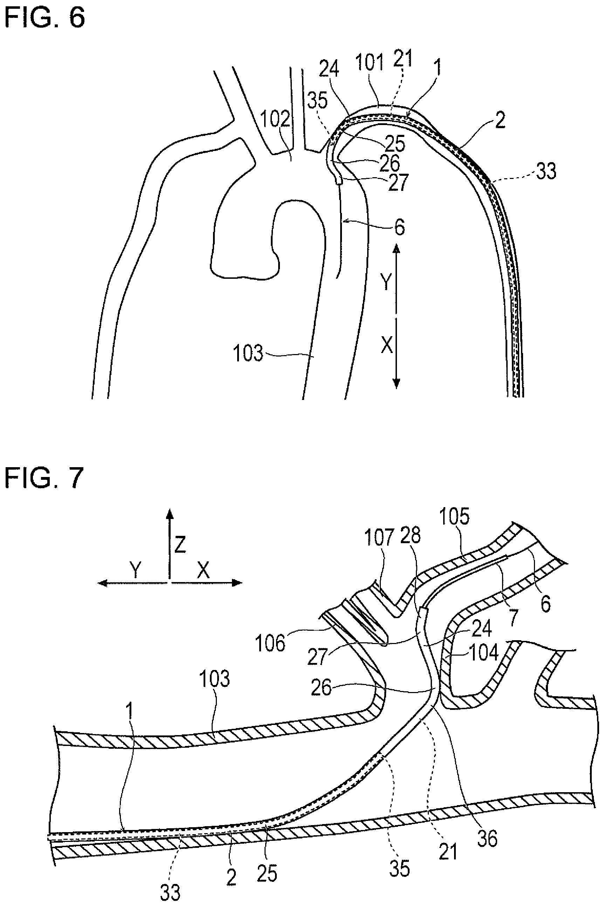

As shown in FIGS. 1 and 7, a catheter 1 according to the present embodiment is a catheter for reaching a common hepatic artery 105. The catheter 1 is introduced into a blood vessel from an artery 100 (for example, a radial artery) of an arm of a patient, and is directed toward vicinity of a hepatic artery. The catheter 1 may be a socalled guiding catheter, an angiographic catheter, a guide wire support catheter, or a microcatheter. In addition, the catheter 1 may be a combination of a guiding catheter and a microcatheter that is inserted into an inner tube of the guiding catheter and is longer than the guiding catheter.

As shown in FIGS. 1 to 3, the catheter 1 includes a tubular body 2, a hub 5 disposed on a proximal side of the tubular body 2, and an strain relief 4.

The tubular body 2 is elongated and flexible. The tubular body 2 is formed with a lumen 21 extending from a proximal end to a distal end in a substantially central portion thereof.

The hub 5 is formed with a passage extending with the lumen 21. The hub 5 can be used to insert or remove, for example, a guide wire 6, a treatment catheter 7, or the like. In addition, the hub 5 can be used to inject various liquids such as an X-ray contrast agent, a drug solution, and a physiological saline solution.

The strain relief 4 is made of an elastic material. The strain relief 4 covers a portion at which the tubular body 2 and the hub 5 are coupled to each other. Accordingly, the strain relief 4 prevents the tubular body 2 from being bent or kink in the vicinity of the portion. Further, a flexible anti-kink tube 8 having an outer diameter larger than that of the tubular body 2 and fixed to the hub 5 into which a proximal side of the tubular body 2 is inserted may be provided between the strain relief 4 and the tubular body 2, or the strain relief 4 may be omitted and the anti-kink tube 8 may be provided alone.

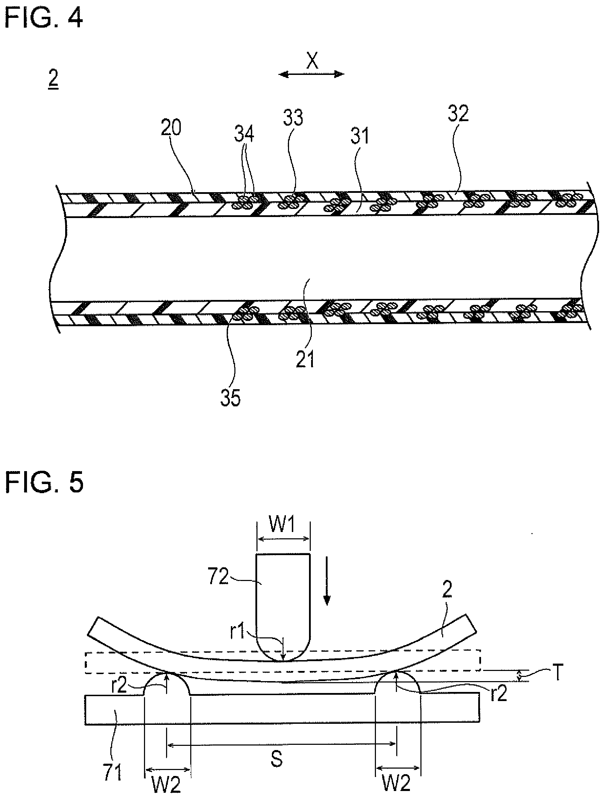

The tubular body 2 will be described in detail. As shown in FIG. 4, the tubular body 2 includes a plurality of layers, and includes an inner layer 31 forming an inner surface of the lumen 21, an outer layer 32 formed outside the inner layer 31, and a reinforcement body 33 embedded in the tubular body 2. Note that a resin of the inner layer 31 and a resin of the outer layer 32 may be the same, or may be different in color, hardness, contrast agent, and the like.

The inner layer 31 includes the lumen 21 formed inside. As a constituent material of the inner layer 31, a thermoplastic resin, a thermosetting resin, or the like can be applied, and a fluorine-based resin such as polytetrafluoroethylene (PTFE), a low-friction material such as high density polyethylene (HDPE), or the like is preferable, and various thermoplastic elastomers such as polyurethane-based, polyester-based, polyamide-based, polybutadiene-based, transpolyisoprene-based, fluororubber-based, and chlorinated polyethylene-based elastomers may be used.

The outer layer 32 is a tubular member that covers an outer periphery of the inner layer 31. The outer layer 32 forms an outer surface radially outward the tubular body 2. Examples of a constituent material of the outer layer 32 include various thermoplastic elastomers such as styrene-based, polyolefin-based, polyurethane-based, polyester-based, polyamide-based, polybutadiene-based, transpolyisoprene-based, fluororubber-based, and chlorinated polyethylene-based elastomers, polyetherketone, and polyimide-based ones, and a combination (polymer alloy, polymer blend, laminate, and the like) of one or two or more of above materials can be used. The outer layer 32 may contain radiopaque material.

The reinforcement body 33 is embedded in the tubular body 2. That is, the reinforcement body 33 is disposed between the inner surface and the outer surface of the tubular body 2. The reinforcement body 33 is formed by braiding a plurality of wires 34 in a tubular shape so as to have gaps. In the reinforcement body 33, the wire 34 may be wound while changing a winding direction, such as transverse winding in the same direction, right winding, left winding, or the like, or a winding pitch, an interlattice distance, an inclination angle with respect to a circumferential direction, or the like may be changed depending on positions, and a configuration is not particularly limited.

A wire diameter (diameter) of the wire 34 of the reinforcement body 33 is not particularly limited, and is, for example, 0.04 mm to 0.05 mm. A cross-sectional shape of the wire 34 is not particularly limited, and is, for example, a circular shape, an elliptical shape, a quadrangular shape, or the like.

As the wire 34 used for the reinforcement body 33, a metal wire such as stainless steel, platinum (Pt), tungsten (W), or the like, a resin fiber, a carbon fiber, a glass fiber, or the like can be applied, or the plurality of these wires 34 may be used in combination.

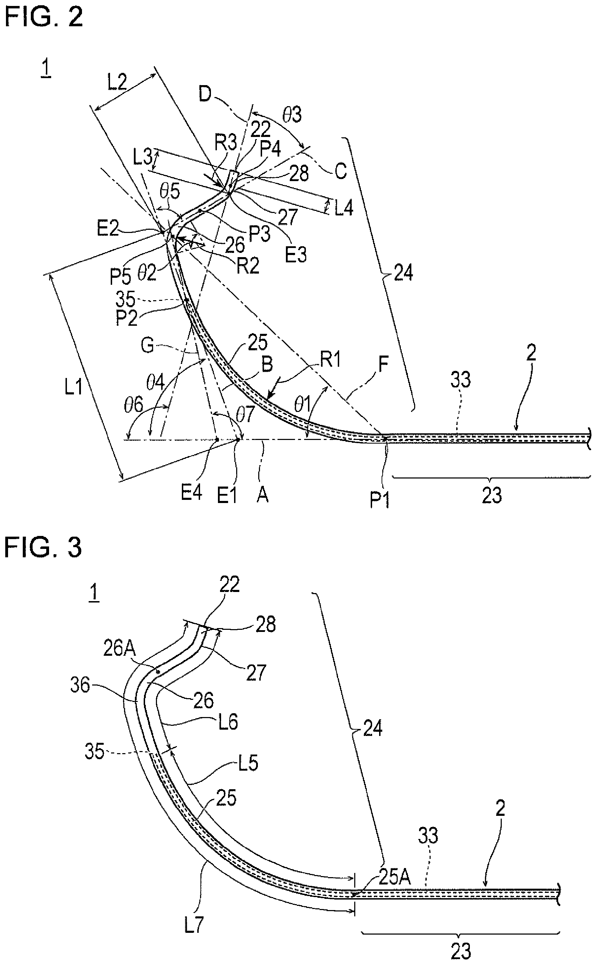

As shown in FIGS. 2 and 3, the tubular body 2 including the inner layer 31, the outer layer 32, and the reinforcement body 33 includes a substantially linear main body portion 23 and a shaped portion 24. The shaped portion 24 is bent on substantially the same plane. The shaped portion 24 may include a linear portion. The shaped portion 24 includes a first bent portion 25, a second bent portion 26, a third bent portion 27, and a most distal portion 28. The first bent portion 25 extends in a distal direction from a first intermediate point P1 located at a distal end of the main body portion 23 and is bent in a plane. The second bent portion 26 extends in the distal direction from a second intermediate point P2 located at a distal end of the first bent portion 25, and is bent in the same direction as the first bent portion 25 in the plane. The second intermediate point P2 is a portion having a largest curvature radius between the first bent portion 25 and the second bent portion 26. When a portion having the largest curvature radius between the first bent portion 25 and the second bent portion 26 has a predetermined range along a center line of the tubular body 2, the second intermediate point P2 can be defined as, for example, a center along the center line of the range. A linear portion may be provided in a predetermined range (length) between the first bent portion 25 and the second bent portion 26. The third bent portion 27 extends in the distal direction from a third intermediate point P3 located at a distal end of the second bent portion 26, and is bent in a direction opposite to the second bent portion 26 in the plane. The third intermediate point P3 is a portion having a largest curvature radius between the second bent portion 26 and the third bent portion 27. When a portion having the largest curvature radius between the second bent portion 26 and the third bent portion 27 has a predetermined range along the center line of the tubular body 2, the third intermediate point P3 can be defined as, for example, a center along a center line of the range. A linear portion may be provided in a predetermined range (length) between the second bent portion 26 and the third bent portion 27. The most distal portion 28 linearly extends in the distal direction from a fourth intermediate point P4 located at a distal end of the third bent portion 27. The fourth intermediate point P4 is located at a proximal end of the most distal portion 28 having a substantially linear shape. Note that the phrase "being curved on substantially the same plane" may include not only being curved on the same plane, but also a case in which a distal portion of the catheter 1 slightly protrudes from the plane to such an extent that the same effect can be practically exhibited.

An axis passing through a center line of the main body portion 23 is defined as a first axis A. A tangent line or a straight line of the center line of the tubular body 2 at the second intermediate point P2 is defined as a second axis B. A tangent line of the center line of the tubular body 2 at the third intermediate point P3 is defined as a third axis C. An axis passing through a center line of the most distal portion 28 is defined as a fourth axis D.

An angle formed by the second axis B on the distal side with respect to the first axis A is defined as a first angle θ4. The first angle θ4 is an amount of change in a direction of the center line of the tubular body 2 in the first bent portion 25. That is, the first bent portion 25 defines the first angle θ4. A curvature radius of the first bent portion 25 may be constant or may be changed in the first bent portion 25. In the first bent portion 25, a first minimum curvature radius R1 of a portion having a smallest curvature radius (a portion bent most sharply) is preferably 10 mm to 80 mm, more preferably 15 mm to 60 mm, and still more preferably 20 mm to 55 mm. In an example of FIG. 2, the first minimum curvature radius R1 is 30 mm. A curvature radius of the second intermediate point P2 is 50 mm. The first angle θ4 is preferably 10° to 120°, more preferably 45° to 90°, and still more preferably 60° to 80°. In the example of FIG. 2, the first angle θ4 is 70°.

An angle formed by the third axis C on the distal side with respect to the second axis B is defined as a second angle θ5. The second angle θ5 is an amount of change in the direction of the center line of the tubular body 2 in the second bent portion 26. That is, the second bent portion 26 defines the second angle θ5. A curvature radius of the second bent portion 26 may be constant or may be changed in the second bent portion 26. In the second bent portion 26, a second minimum curvature radius R2 of a portion having a smallest curvature radius (a portion bent most sharply) is preferably 5 mm to 15 mm, more preferably 7 mm to 12 mm, and still more preferably 8 mm to 11 mm. In the example of FIG. 2, the second minimum curvature radius R2 is 9 mm. The second angle θ5 is preferably 10° to 170°, more preferably 55° to 100°, and still more preferably 70° to 90°. In the example of FIG. 2, the second angle θ5 is 80°. The second minimum curvature radius R2 is smaller than the first minimum curvature radius R1.

An angle formed by the fourth axis D on the distal side with respect to the third axis C is defined as a third angle θ3. The third angle θ3 is an amount of change in the direction of the center line of the tubular body 2 in the third bent portion 27. That is, the third bent portion 27 defines the third angle θ3. A curvature radius of the third bent portion 27 may be constant or may be changed in the third bent portion 27. In the third bent portion 27, a third minimum curvature radius R3 of a portion having a smallest curvature radius (a portion bent most sharply) is preferably 1 mm to 9 mm, more preferably 2 mm to 8 mm, and still more preferably 4 mm to 7 mm. In the example of FIG. 2, the third minimum curvature radius R3 is 5 mm. The third angle θ3 is preferably more than 0° and 90° or less, more preferably 20° to 70°, and still more preferably 30° to 60°. In the example of FIG. 2, the third angle θ3 is 45° .

An intersection point of the first axis A and the second axis B is defined as a first intersection point E1, an intersection point of the second axis B and the third axis C is defined as a second intersection point E2, and an intersection point of the third axis C and the fourth axis D is defined as a third intersection point E3.

A distance L1 between the first intersection point E1 and the second intersection point E2 is preferably 5 mm to 100 mm, more preferably 20 mm to 60 mm, and still more preferably 30 mm to 50 mm. In the example of FIG. 2, the distance L1 is 34 mm.

A distance L2 between the second intersection point E2 and the third intersection point E3 is preferably 1 mm to 80 mm, more preferably 5 mm to 50 mm, and still more preferably 10 mm to 2 mm. In the example of FIG. 2, the distance L2 is 12 mm.

A distance L3 between the third intersection point E3 and a most distal end of the most distal portion 28 is preferably 0.1 mm to 60 mm, more preferably 1 mm to 40 mm, and still more preferably 2 mm to 30 mm. In the example of FIG. 2, the distance L3 is 3.5 mm.

A length L4 along the center line of the most distal portion 28 is preferably 0.1 mm to 50 mm, more preferably 0.5 mm to 30 mm, and still more preferably 1 mm to 20 mm. In the example of FIG. 2, the length L4 is 1.4 mm.

In the second bent portion 26, the portion having the smallest curvature radius (the portion bent most sharply) is defined as a second bent point P5. In the second bent portion 26, when the portion having the smallest curvature radius has a predetermined range along the center line of the tubular body 2, the second bent point P5 can be defined as, for example, the center along the center line of the range. An axis passing through the second bent point P5 and the first intermediate point P1 located at the distal end of the main body portion 23 is defined as a fifth axis F. An angle formed by the fifth axis F on the distal side with respect to the first axis A is defined as a first inclination angle θ1. The first inclination angle is preferably 10° to 110°, more preferably 34° to 63°, and still more preferably 40° to 50°. In the example of FIG. 2, the first inclination angle θ1 is 46°. The larger the first inclination angle θ1 is, the larger bending of the tubular body 2 is.

A second inclination angle θ2 is an angular range having the second minimum curvature radius R2 in the second bent portion 26. That is, the second bent portion 26 has the second minimum curvature radius R2 in the range of the second inclination angle θ2. The second inclination angle θ2 is preferably 5° to 120°, more preferably 10° to 90°, and still more preferably 38° to 80°. In the example of FIG. 2, the second inclination angle θ2 is 73°.

An angle formed by the fourth axis D on the distal side with respect to the first axis A is defined as a third inclination angle θ6. The third inclination angle θ6 is an angle formed by the most distal portion 28 and the main body portion 23. The third inclination angle θ6 is preferably 0° to 240° or less, more preferably 80° to 150°, and still more preferably 90° to 120°. In the example of FIG. 2, the third inclination angle θ6 is 105°.

Alternatively, the curvature radius of the second intermediate point P2 may be 40 mm or more, and more preferably 50 mm or more. Still more preferably, the second intermediate point P2 may be a substantially straight line. Further, a direction in which a straight line G connecting the second intermediate point P2 and the second bent point P5 extends from the second intermediate point P2 toward the second bent point P5 is a direction away from the main body portion 23, and is a direction away from the hub 3 in an extending direction of the main body portion 23. In other words, an angle θ7 formed by the second intermediate point P2, an intersection point E4 between the straight line G and the first axis A, and the first intermediate point P1 is an obtuse angle. In this case, the catheter 1 can be prevented from entering a left gastric artery 106 or a splenic artery 107. In the example of FIG. 2, θ7 is 102°.

In the tubular body 2, the reinforcement body 33 is embedded in a range from the proximal end to a predetermined position in the distal direction. A distal end 35 of the reinforcement body 33 is disposed between a proximal portion 25A of the first bent portion 25 and a distal portion 26A of the second bent portion 26. In the present embodiment, the distal end 35 of the reinforcement body 33 is disposed between the first bent portion 25 and the second bent portion 26.

The catheter 1 may be coated with a lubricating coating in the proximal direction along an axis from the distal end opening portion 22. The lubricating coating may be coated in the entire length, and is coated in, for example, a range of at least 400 mm or less, more preferably 200 mm or less in the proximal direction along the axis from the distal end opening portion 22. Therefore, the surgeon can deeply insert the catheter 1 into the common hepatic artery 105. Examples of the constituent material of the lubricating coating include: a copolymer including an epoxy group-containing monomer such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether, and a hydrophilic monomer such as N-methylacrylamide, N,N-dimethylacrylamide, and acrylamide; a (co)polymer including the hydrophilic monomer; a cellulose-based polymer material such as hydroxypropyl cellulose and carboxymethyl cellulose; a polysaccharide; a polyvinyl alcohol; a methyl vinyl ethermaleic anhydride copolymer; a water-soluble polyamide; poly(2-hydroxyethyl (meth)acrylate) ; polyethylene glycol; polyacrylamide; and polyvinyl pyrrolidone.

An outer diameter of the tubular body 2 is preferably 1 mm (3 Fr) to 2.5 mm (6 Fr), and more preferably 1.3 mm (4 Fr) to 1.8 mm (5 Fr) . An effective length of the tubular body 2 is preferably 800 mm to 1800 mm, more preferably 1000 mm to 1800 mm, and still more preferably 1000 mm to 1500 mm, and may be yet more preferably selected from 1100 mm, 1200 mm, 1250 mm, 1300 mm depending on a physique or for a reason of insertion from a distal radial artery or a snuff box radial artery. It is preferable that the tubular body 2 can be appropriately selected depending on the patient, a blood vessel to which the catheter 1 is to be introduced, or the like. For example, when the catheter 1 is introduced from a brachial artery of a small-sized female, the effective length of the tubular body 2 is preferably relatively short. When the catheter 1 is introduced from a distal radial artery of a large-sized male, the effective length of the tubular body 2 is preferably relatively long. The effective length is a length of a portion that can be inserted into a blood vessel, a sheath, or the like. In the present embodiment, the effective length is a length from a most distal end of the strain relief 4 to a most distal end of the tubular body 2.

A length L5 along the central axis of the tubular body 2 from the proximal portion 25A of the first bent portion 25 of the tubular body 2 to the distal end 35 of the reinforcement body 33 is preferably 1 mm to 75 mm, and more preferably 20 mm to 45 mm. A length L6 along the central axis of the tubular body 2 from the distal end 35 of the reinforcement body 33 of the tubular body 2 to a distal end of the tubular body 2 is preferably 1 mm to 50 mm, and more preferably 20 mm to 30 mm. Note that a length along the central axis of the tubular body 2 is equal to a length when the tubular body 2 is deformed linearly. A length L7 from the proximal portion 25A of the first bent portion 25 of the tubular body 2 to the distal end of the tubular body 2 is a sum of the length L5 and the length L6 described above, and is preferably 40 mm to 75 mm, and more preferably 50 mm to 75 mm.

A ratio of L6/L7 is preferably 1% to 100%, more preferably 10% to 60%, and still more preferably 10% to 40%. Alternatively, a ratio of L6/L5 is preferably 1% to 200%, and more preferably 10% or more and less than 70%. For example, L5 is 37 mm, L6 is 23 mm, L7 is 60 mm, L6/L7 is 38%, and L6/L5 is 62%.

The tubular body 2 includes a flexible portion 36 in which no reinforcement body 33 is provided distal of a portion in which the reinforcement body 33 is embedded. Since the catheter 1 includes the flexible portion 36, the catheter 1 can move even in a bent or bifurcated blood vessel without damaging the blood vessel. A length of the flexible portion 36 is equal to the aforementioned length L6. In a case of a catheter 1 of 4 Fr, the length L6 of the flexible portion 36 is 23 mm. In a case of a catheter 1 of 5 Fr, the length L6 of the flexible portion 36 is 23 mm. The flexible portion 36 has a bending characteristic lower than that of the portion in which the reinforcement body 33 is provided. The bending characteristic may be a bending elastic modulus, an actual measurement value of a load or a stress by a three-point bending test, or the like. A bending elastic modulus of at least a part of the tubular body 2 is preferably 2 MPa to 25 MPa, more preferably 4 MPa to 20 MPa, still more preferably 7 MPa to 13 MPa, and yet still more preferably 9 MPa to 10 MPa. A load of the portion of the tubular body 2 in which the reinforcement body 33 is provided obtained by the three-point bending test is preferably 20 gf to 250 gf, and more preferably 40 gf to 120 gf. When the outer diameter of the catheter 1 is 4 Fr, the bending characteristic (load) is preferably 40 gf or more. When the outer diameter of the catheter 1 is 5 Fr, the bending characteristic (load) is preferably 80 gf or more.

An example of measurement of the bending characteristic by the three-point bending test is shown in FIG. 5. As measurement conditions, for example, a room temperature is 25°C, a distance S between supporting points of a support table 71 is 25.4 mm (1 inch), a test speed of an indenter 72 pressing a center position between the supporting points from an opposite side is 5 mm/min, and a pressing amount T of the indenter 72 is 2 mm. An indenter width W1 is 1.0 mm, and a curvature radius r1 of the indenter is 0.5 mm. A supporting point width W2 of each supporting point 73 of the support table 71 is 4.0 mm, and a curvature radius r2 of each supporting point 73 is 0.5 mm. A pressing load measured under the conditions can be used as the bending characteristic.

A distal tip that is more flexible than the proximal side may be disposed at the distal portion of the tubular body 2. The distal tip is made of a highly flexible material such as a rubber material.

The number of layers constituting the tubular body 2, a constituent material of each layer, presence or absence of the reinforcement body, and the like may be different in a longitudinal direction of the tubular body 2.

Next, a method of using the catheter 1 according to the present embodiment will be described.

As shown in FIG. 1, the catheter 1 is inserted into the blood vessel from the artery 100 of the arm, and is engaged with a celiac artery 104. The artery 100 of the arm into which the catheter 1 is to be introduced is, for example, a distal radial artery, a conventional radial artery, an ulnar artery, a distal ulnar artery, a brachial artery, a snuff box radial artery, or the like. Here, for example, a case in which the catheter 1 is inserted into an artery of a left arm will be described. Note that the catheter 1 may be inserted from an artery of a right arm.

In a procedure, the surgeon inserts the guide wire 6 into the artery 100 of the arm. Next, the surgeon pushes the catheter 1 in which the guide wire 6 is accommodated in the lumen 21 forward along the guide wire 6. Normally, a distal end of the guide wire 6 precedes the distal end of the catheter 1. Therefore, the shaped portion 24 of the catheter 1 is deformed by the guide wire 6 in the lumen 21 into a shape closer to a straight line.

As shown in FIG. 6, the guide wire 6 and the catheter 1 pass through the subclavian artery 101 and advance to an aortic arch 102. At a portion at which the subclavian artery 101 is connected to the aortic arch 102, the catheter 1 needs to be bent greatly in order to move toward the descending aorta 103. In order to cope with this need, for example, the surgeon can temporarily retract the guide wire 6 and accommodate the guide wire 6 in the lumen 21 of the catheter 1. Accordingly, the shaped portion 24 of the catheter 1 is restored to an original bent shape. The surgeon can advance the distal end of the catheter 1 from the aortic arch 102 toward the descending aorta 103 by using bending of the shaped portion 24. Since the shaped portion 24 is provided with the reinforcement body 33 and thus can easily maintain the shape thereof, the catheter 1 can easily access the descending aorta 103 from the subclavian artery 101. Thereafter, the surgeon causes the guide wire 6 to protrude from the catheter 1. Accordingly, the guide wire 6 can easily advance to the descending aorta 103. Subsequently, the surgeon pushes the catheter 1 forward along the guide wire 6. Accordingly, the catheter 1 can easily advance from the aortic arch 102 to the descending aorta 103. The catheter 1 moves to a lower side X (a side close to a lower limb) in the descending aorta 103 and reaches the vicinity of the celiac artery 104.

After the guide wire 6 and the catheter 1 reach the vicinity of an entrance of the celiac artery 104, the surgeon retracts the guide wire 6 to accommodate the guide wire 6 in the lumen 21 of the catheter 1. Accordingly, as shown in FIG. 7, the shaped portion 24 of the catheter 1 is restored to the original bent shape. The surgeon can use the bending of the shaped portion 24 to insert the distal end of the catheter 1 into the celiac artery 104. The celiac artery 104 extends from the descending aorta 103 generally toward a front side Z (celiac side). The common hepatic artery 105, the left gastric artery 106, and the splenic artery 107 are bifurcated from the celiac artery 104. The common hepatic artery 105 generally extends from the celiac artery 104 to the lower side X (the side close to the lower limb) . The left gastric artery 106 and the splenic artery 107 generally extend from the celiac artery 104 to an upper side Y (a side close to a head) . The second bent portion 26 of the catheter 1 is in contact with a blood vessel wall of the celiac artery 104, and the first bent portion 25 is in contact with a blood vessel wall of the descending aorta 103 on a side opposite to the entrance of the celiac artery 104. The second bent portion 26 is in contact with the blood vessel wall of the celiac artery 104 on the lower side X. Accordingly, the second bent portion 26 of the catheter 1 is engaged with the celiac artery 104. In this state, the third bent portion 27 is bent to a side opposite to the first bent portion 25 and the second bent portion 26. Therefore, a most distal end of the catheter 1 is likely to be directed to the common hepatic artery 105 extending from the celiac artery 104 to the lower side X. The third bent portion 27 and/or the most distal portion 28 may or may not be in contact with the blood vessel wall of the common hepatic artery 105.

In the catheter 1, since the distal end 35 of the reinforcement body 33, which is a physical property changing point, is disposed at a predetermined distance (for example, 20 mm to 30 mm) from the distal end, the flexible portion 36 distal of the reinforcement body 33 comes into contact with a wall surface of the celiac artery 104, thereby stabilizing a position thereof. Furthermore, since the reinforcement body 35 is not provided in the flexible portion 36, a degree of freedom of blood vessel selection in the celiac artery 104 having many branches and bends is increased, or the blood vessel selection by the guide wire 6 is facilitated. Note that in a guiding catheter for a coronary artery, since a distal bent portion to be hooked on an entrance of the coronary artery is disposed on an aorta side, a reinforcement body is generally provided up to the vicinity of a soft tip disposed at 2 mm to 3 mm of the distal end, that is, up to the distal bent portion. In contrast, the catheter 1 according to the present embodiment is for the hepatic artery, and at least the second bent portion 26 is inserted into the celiac artery 104, and therefore a structure and an engagement method are different from those of the guiding catheter for the coronary artery. Further, unlike a catheter of Jacky type or Sarah type in which a bent portion protrudes by being bent sharply, since the first bent portion 25 is smoothly bent, the first bent portion 25 can be smoothly brought into contact with the blood vessel wall, and a burden on the blood vessel can be reduced.

Since a catheter that is to be inserted from a femoral artery and engaged with the celiac artery 104 is disposed substantially linearly from the femoral artery to the celiac artery 104 in the related art, a backup force can be exerted even if no reinforcement body 33 is provided in a shaped portion. However, when the catheter of the related art is introduced from an artery of an arm, "prolapse" in which a part of the catheter bends toward an ascending aorta opposite to the descending aorta 103 due to injection of a contrast agent or an operation of a microcatheter may occur.

In order to prevent the prolapse, the end portion 35 (physical property changing point) of the reinforcement body 33 of the catheter 1 is preferably provided between the proximal portion 25A of the first bent portion 25 and the distal portion 26A of the second bent portion 26. Flexural rigidity of the catheter 1 greatly changes at the physical property changing point and decreases toward the distal side. In addition to the end portion 35 of a reinforcement body 33, the physical property changing point may be changed such that a hardness of the resin of the outer layer 32 and/or the inner layer 31 is more flexible toward the distal side, or may be provided such that the catheter 1 is thinner toward the distal side.

Further, in order to further increase a repulsive force of the catheter 1, the end portion 35 of the reinforcement body 33 may be provided in the vicinity of the second intermediate point P2 which has a maximum curvature radius, or may be made to coincide with the second intermediate point P2.

Thereafter, the treatment catheter 7, which is longer than the catheter 1, is inserted into the lumen 21 of the catheter 1 from the hub 5. The surgeon can easily insert the treatment catheter 7 into the common hepatic artery 105 by protruding the treatment catheter 7 from the distal end of the catheter 1. At this time, since the most distal end of the catheter 1 is directed from the celiac artery 104 to the lower side X, it is possible to prevent the treatment catheter 7 from being erroneously inserted into the left gastric artery 106 or the splenic artery 107 directed to the upper side Y. Thereafter, the surgeon inserts the treatment catheter 7 into the common hepatic artery 105, and can release an embolic agent or a drug solution via the treatment catheter 7. The catheter 1 relaxes a reaction generated by the insertion of the treatment catheter 7 and the guide wire 6. Further, the catheter 1 can apply the backup force to the treatment catheter 7 to hold the treatment catheter 7 at a desired position. Note that a medical device to be inserted into the catheter 1 may not be the treatment catheter 7.

As described above, the catheter 1 according to the present embodiment is the catheter 1 for the hepatic artery to be introduced from the arm of the patient. The catheter 1 includes the tubular body 2 extending from the proximal end to the distal end. The tubular body 2 includes the inner layer 31, the outer layer 32, and the reinforcement body 33 embedded in the tubular body 2. The tubular body 2 includes the substantially linear main body portion 23 and the shaped portion 24 shaped in a manner of being bent on substantially the same plane. The shaped portion 24 includes the first bent portion 25 defining the first angle θ4 distal of the main body portion 23, the second bent portion 26 defining the second angle θ5 distal of the first bent portion 25 and bent to the same side as the first bent portion 25, the third bent portion 27 defining the third angle θ3 distal of the second bent portion 26 and bent to a side opposite to the second bent portion 26, and the most distal portion 28 disposed distal of the third bent portion 27. The physical property changing point is provided between the proximal portion 25A of the first bent portion 25 and the distal portion 26A of the second bent portion 26.

In the catheter 1 configured as described above, since the physical property changing point is disposed between the proximal portion 25A of the first bent portion 25 and the distal portion 26A of the second bent portion 26, the position of the shaped portion 24 of the catheter 1 is stabilized by the first bent portion 25 which has a high strength coming into contact with the descending aorta 103. Since the second bent portion 26 which is at least partially flexible is engaged with the celiac artery 104 in a favorable manner, and the flexible third bent portion 27 disposed distal of the physical property changing point is bent to a side opposite to the second bent portion 26, the most distal portion 28 is easily directed to the common hepatic artery 105. Therefore, the catheter 1 can apply the favorable backup force to another medical device such as the treatment catheter 7 and the guide wire 6 inserted into the catheter 1. Further, since the shaped portion 24 provided with the physical property changing point is likely to firmly maintain the shape proximal of the physical property changing point, an access of the catheter 1 from the subclavian artery 101 (left subclavian artery or right subclavian artery) to the descending aorta 103 is facilitated. Therefore, operability of the catheter 1 is improved.

The physical property changing point is disposed between the first bent portion 25 and the second bent portion 26. Accordingly, the distal side of the catheter 1 can be stably directed toward the celiac artery 104 by the bending of the first bent portion 25 which has a high strength, and the second bent portion 26 which is flexible and disposed distal of the physical property changing point can be deformed in accordance with the celiac artery 104 to be deeply engaged with the celiac artery 104 (deep engage). Therefore, the catheter 1 can apply the favorable backup force to another medical device such as the treatment catheter 7 and the guide wire 6 inserted into the catheter 1. Since the second bent portion 26 is flexible, it is possible to reduce the burden on the blood vessel wall in contact therewith. Further, since a strength of the entire first bent portion 25 is improved, a range in which the shape of the shaped portion 24 can be firmly maintained is widened, and the access of the catheter 1 from the subclavian artery 101 to the descending aorta 103 is facilitated.

The physical property changing point is the end portion 35 of the reinforcement body 33. Since the reinforcement body 33 enhances the hardness, it is possible to easily provide the physical property changing point without changing the resin of the inner layer 31 and the outer layer 32.

When the second bent portion 26 is disposed in contact with the blood vessel wall of the celiac artery 104, the most distal portion 28 is directed to the common hepatic artery 105, and the first bent portion 25 is in contact with a wall surface of the descending aorta 103 on a side opposite to the celiac artery 104. Accordingly, the second bent portion 26 can be engaged with the celiac artery 104 in a favorable manner in a state in which the first bent portion 25 which has a high strength is stably brought into contact with the blood vessel wall of the descending aorta 103 on the side opposite to the celiac artery 104.

The invention further includes a method for engagement of the catheter 1. The method for engagement of the catheter 1 includes: a step of inserting the catheter 1 including the physical property changing point between the proximal portion 25A of the first bent portion 25 and the distal portion 26A of the second bent portion 26 into the artery of the arm (for example, the radial artery 100); a step of advancing the shaped portion 24 of the catheter 1 to a main artery accessible from the artery of the arm (for example, the descending aorta 103); a step of advancing the shaped portion 24 to a first blood vessel (for example, the celiac artery 104) coupled in a direction intersecting with a long axis of the main artery; and a step of bringing the second bent portion 26 into contact with a blood vessel wall of the first blood vessel and attaching the first bent portion 25 to a blood vessel wall of the main artery on a side opposite to the first blood vessel to direct the most distal portion 28 to a second blood vessel selected from at least two blood vessels bifurcated on a peripheral side of the first blood vessel.

In the method for catheter 1 engagement configured as described above, the position of the shaped portion 24 of the catheter 1 can be stabilized by bringing the first bent portion 25 which has a high strength into contact with the main artery by the reinforcement body 33. Then, the second bent portion 26 which is at least partially flexible can be engaged with the first blood vessel in a favorable manner, and the most distal portion 28 can be directed toward the second blood vessel. Therefore, the surgeon can easily insert another medical device into the second blood vessel using the catheter 1 as a guide.

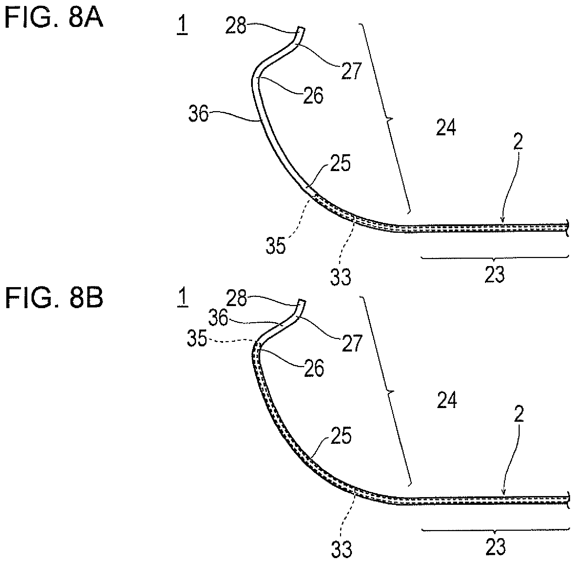

Note that the invention is not limited to the embodiment described above, and various modifications can be made by those skilled in the art within a scope of the technical idea of the invention. For example, the catheter 1 may be appropriately designed within the ranges of various angles, dimensions, and the like described above. For example, as in a first modification shown in (A) in FIG. 8, the end portion 35 of the reinforcement body 33, which is a physical property changing point, may be disposed in the first bent portion 25. Accordingly, a distal side of the catheter 1 can be stably directed toward the celiac artery 104 by bending of the first bent portion 25 which includes the reinforcement body 33 and has a high strength, and the flexible second bent portion 26 which is disposed distal of the reinforcement body 33 can be deformed in accordance with the celiac artery 104 to be deeply engaged with the celiac artery 104. Therefore, the catheter 1 can apply a favorable backup force to another medical device inserted into the catheter 1. Further, the second bent portion 26 is flexible, and thus can reduce a burden on a blood vessel wall in contact therewith.

In addition, as in a second modification shown in (B) in FIG. 8, the end portion 35 of the reinforcement body 33, which is a physical property changing point, may be disposed in the second bent portion 26. Accordingly, since strengths of the first bent portion 25 and the second bent portion 26 are improved, a range in which a shape of the shaped portion 24 can be firmly maintained is widened, and an access of the catheter 1 from the subclavian artery 101 to the descending aorta 103 is facilitated.

Hereinafter, specific examples of the invention will be described in detail.

### Example 1

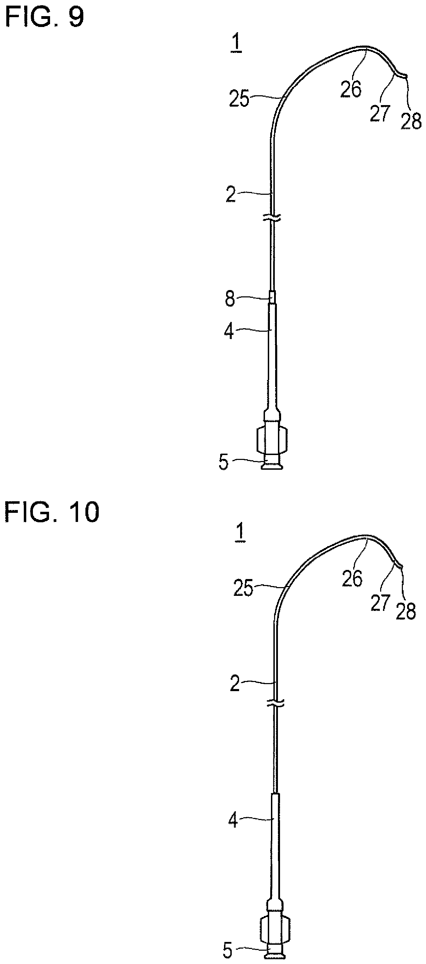

The catheter 1 including the anti-kink tube 8 shown in FIG. 9 was produced. Parameters of the catheter 1 were as follows.
Effective length: 1250 mm
Outer diameter: 1.4 mm (4 Fr)
Inner diameter (lumen diameter): 1.1 mm
Cross-sectional structure: laminate of inner layer, reinforcement body, and outer layer
Reinforcement wire diameter and structure: 0.04 mm, double mesh wire
Distal lubricating coating length: 150 mm
θ1: 47°
θ2: 77°
θ3: 45°
θ4: 70°
θ5: 85°
θ6: 102°
θ7: 97°
L1: 34 mm
L2: 12 mm
L3: 3.5 mm
L4: 1.4 mm
L5: 37 mm
L6: 23 mm
L7: 60 mm
R1: 30 mm
R2 : 9 mm
R3: 5 mm
Curvature radius at P2: 50 mm

### Example 2

The catheter 1 shown in FIG. 10 was produced. Parameters of the catheter 1 were as follows.
Effective length: 1250 mm
Outer diameter: 1.7 mm (5 Fr)
Inner diameter (lumen diameter): 1.2 mm
Cross-sectional structure: laminate of inner layer, reinforcement body, and outer layer
Reinforcement wire diameter and structure: 0.05 mm, double mesh wire
Distal lubricating coating length: 150 mm
θ1: 44°
θ2: 76°
θ3: 20°
θ4: 70°
θ5: 81°
θ6: 129°
θ7: 99°
L1: 34 mm
L2: 12 mm
L3: 3.5 mm
L4: 1.4 mm
L5: 37 mm
L6: 23 mm
L7: 60 mm
R1: 30 mm
R2: 9 mm
R3: 5 mm
Curvature radius at P2: 50 mm

Note that a blood vessel with which the catheter 1 is to be engaged is not limited to the celiac artery 104. For example, the blood vessel with which the catheter 1 is to be engaged may be a superior mesenteric artery, an inferior mesenteric artery, a renal artery, a lumbar artery, a splenic artery, a left gastric artery, a testicular artery, a common iliac artery, an internal iliac artery, a prostate artery, a uterine artery, an arteria iliaca externa, a coronary artery, or the like.

Note that the present application is based on Japanese Patent Application No. 2020-131419 filed on August 3, 2020 and Japanese Patent Application No. 2020-197871 filed on November 30, 2020, and the disclosure thereof is referenced and incorporated as a whole.

### Reference Signs List

1 catheter
2 tubular body
6 guide wire
7 treatment catheter
8 anti-kink tube
21 lumen
23 main body portion
24 shaped portion
25 first bent portion
26 second bent portion
27 third bent portion
28 most distal portion
31 inner layer
32 outer layer
33 reinforcement body
34 wire rod
35 distal end of reinforcement body
36 flexible portion
100 radial artery
101 subclavian artery
102 aortic arch
103 descending aorta
104 celiac artery
105 common hepatic artery
106 left gastric artery
107 splenic artery

## Claims

1. A catheter for a hepatic artery to be introduced from an arm of a patient, the catheter comprising:
a tubular body extending from a proximal end to a distal end,
wherein the tubular body includes an inner layer, an outer layer, and a reinforcement body embedded in the tubular body,
the tubular body includes a substantially linear main body portion and a shaped portion shaped in a manner of being bent on substantially the same plane,
the shaped portion includes:
a first bent portion defining a first angle distal of the main body portion;
a second bent portion defining a second angle distal of the first bent portion and bent to the same side as the first bent portion;
a third bent portion defining a third angle distal of the second bent portion and bent to a side opposite to the second bent portion; and
a most distal portion disposed distal of the third bent portion, and
a physical property changing point is provided between a proximal portion of the first bent portion and a distal portion of the second bent portion.

2. The catheter according to claim 1,
wherein the physical property changing point is disposed in the first bent portion.

3. The catheter according to claim 1,
wherein the physical property changing point is disposed in the second bent portion.

4. The catheter according to claim 1,
wherein the physical property changing point is disposed between the first bent portion and the second bent portion.

5. The catheter according to any one of claims 1 to 4,
wherein the physical property changing point is an end portion of the reinforcement body.

6. The catheter according to any one of claims 1 to 5,
wherein when the second bent portion is disposed in contact with a blood vessel wall of a celiac artery, the most distal portion is directed to a common hepatic artery, and the first bent portion is in contact with a wall surface of a descending aorta on a side opposite to the celiac artery.

7. A method for catheter engagement comprising:
a step of inserting, into an artery of an arm of a patient, a catheter including a tubular body extending from a proximal end to a distal end, the tubular body including an inner layer, an outer layer, and a reinforcement body embedded in the tubular body, the tubular body including a substantially linear main body portion and a shaped portion shaped in a manner of being bent on substantially the same plane, the shaped portion including a first bent portion disposed distal of the main body portion, a second bent portion disposed distal of the first bent portion and bent to the same side as the first bent portion, a third bent portion disposed distal of the second bent portion and bent to a side opposite to the second bent portion, and a most distal portion disposed distal of the third bent portion, and a physical property changing point being provided between a proximal portion of the first bent portion and a distal portion of the second bent portion;
a step of advancing the shaped portion of the catheter to a main artery accessible from the artery of the arm;
a step of advancing the shaped portion to a first blood vessel coupled in a direction intersecting a long axis of the main artery; and
a step of bringing the second bent portion into contact with a blood vessel wall of the first blood vessel and attaching the first bent portion to a blood vessel wall of the main artery on a side opposite to the first blood vessel to direct the most distal portion to a second blood vessel selected from at least two blood vessels bifurcated on a peripheral side of the first blood vessel.

8. The method for catheter engagement according to claim 7,
wherein the physical property changing point is an end portion of the reinforcement body.
